# EUROPEAN PATENT APPLICATION

(11) **EP 3 171 177 A1**
(43) Date of publication of application: **24.05.2017**
(21) Application number: 15195307.2
(22) Date of filing: 19.11.2015
(51) Int. Cl.: G01N 35/00, G01N 35/10

(54) **METHOD AND LABORATORY SYSTEM FOR DISTRIBUTING BIOLOGICAL SAMPLES ONTO MICROPLATES**

(71) Applicant: F. Hoffmann-La Roche AG, 4070 Basel (CH); Roche Diagnostics GmbH, 68305 Mannheim (DE)
(72) Inventor: Koetting, Ewald, 14482 Postdam (DE); Tietze, Heiko, 10557 Berlin (DE)
(74) Representative: Gyenge, Zoltán

(57) **Abstract**

Disclosed is a method and a system configured to carry out a method for distributing one or more biological samples to a plurality of wells (3) of a microplate (1), the method comprising: retrieving (102) an allocation template (10) corresponding to the microplate (1); selecting (105) a sample distribution strategy (50) from one or more sample distribution strategies; retrieving (103) a test order list (30) corresponding to the one or more biological samples; generating (107) a sample distribution list (70) assigning the one or more biological samples to the plurality of wells (3) of the microplate (1) according to the test order list (30), the allocation template (10) and the selected sample distribution strategy (50); and distributing (115) the one or more biological samples onto the plurality of wells (3) of the microplate (1) according to the sample distribution list (70).

## Description

### TECHNICAL FIELD

The disclosed method/ system relate to a method and an automated laboratory system for distributing one or more biological samples to a plurality of wells of a microplate.

### BACKGROUND

Microplates or microwell plates are commonly used in many analytical research and clinical diagnostic testing laboratories. Each well of a microplate typically holds somewhere between tens of nanolitres to several milliliters of biological sample. Microplates have various dimensions, formats and configurations. Common microplates have 6, 24, 96, 384 or even 1536 sample wells arranged in a rectangular matrix. Furthermore, microplates have several varying characteristics, such as allocation sequence, assignment restrictions, etc. Colorimetric immunoassays are widely used, the most common type being an ELISA (enzyme-linked immunosorbent assay) method. In colorimetric immunoassays, a source of incident light is directed onto or through the assay reagents (typically in a clear microplate). Other photometric assay techniques performed in microplate plates are termed photon emitting immunoassays. These include chemiluminescent, bioluminescent and fluorescent immunoassays.

Before performing analytical testing using a microplate, one or more biological samples have to be distributed into one or more of the wells of the microplate. However, the distribution of the biological samples onto microplates is usually a time-consuming process. Laboratory personnel has to select the biological samples according several prerequisites, such as schedule, priority of the corresponding test orders, completeness, and assign those samples to wells of the microplate in respect to the type of analysis, capability of the analytical instrument and/or laboratory practice.

Although prior art shows the existence of various pipetting systems for microplates, there is still no efficient solution for distributing samples onto different microplates which would adapt to the needs of a variety of laboratory processes.

### SUMMARY

Embodiments of the disclosed method/ device aim at improving the process of distributing biological samples onto wells of microplate(s) by applying a sample distribution strategy of a test order list on an allocation template corresponding to the microplate. The allocation template(s) define particularities of the microplate (such as but not limited to physical dimensions, format, allocation sequence, assignment restrictions, etc.) while the sample distribution strategies are defined taking into account test types and/or workflow particularities of a laboratory. Therefore, according to embodiments herein disclosed, the sample distribution is a multi-step process including the selection of test orders from a test order list according to a sample distribution strategy followed by the assignment of these items to the right position on the microplate based on the corresponding allocation template.

Embodiments of the disclosed method/ device are advantageous as the occurrence of errors in the sample distribution process can be minimized / avoided as the distribution of the samples is performed according to an allocation template corresponding to a particular microplate.

Embodiments of the disclosed method/ device are advantageous as the sample distribution process of a laboratory can be optimized based on specific requirements of the particular laboratory. Depending on the selected sample distribution strategy, the distribution can be optimized to prioritize test orders of the same patient so that analytical tests of a patient are distributed - and eventually performed - as soon as possible. Alternatively or additionally, the distribution can be optimized to prioritize test orders for the same analytical test (same analyte) so that consumables - in particular microplates - are utilized as effectively as possible. Alternatively or additionally, the distribution can be optimized to group test orders with the same origin (such as same laboratory, same clinic, etc.). Alternatively or additionally sample distribution strategies can be designed to achieve a balance between these advantages.

Embodiments of the disclosed method/ device are advantageous as generating a distribution list according to a distribution strategy and specific templates for the microplates allows determining / estimating the number of disposables - in particular the number of microplates - required for processing a test order list.

Further embodiments of the disclosed method/ device are advantageous as a user interface can be provided which allows a what you see is what you get WYSIWYG visualisation of allocation templates before and after distribution of the samples into the wells of the microplate.

### BRIEF DESCRIPTION OF THE DRAWINGS

Further characteristics and advantages of the disclosed method/ device/ system will in the following be described in detail by means of the description and by making reference to the drawings. Which show:
- Fig. 1: a flowchart illustrating embodiments of a method for distributing one or more biological samples to a plurality of wells of a microplate;
- Fig. 2: a particular embodiment of a microplate with a plurality of wells arranged in a number of rows and columns;
- Fig. 3A: a particular embodiment of an allocation template illustrating a particular allocation sequence and particular assignment restrictions comprising dedicated wells for a particular analyte;
- Fig. 3B: a further particular embodiment of an allocation template illustrating a particular allocation sequence and particular assignment restrictions comprising dedicated wells for a particular analyte as well as reserved wells for control materials;
- Fig. 3C: a further particular embodiment of an allocation template illustrating a particular allocation sequence and particular assignment restrictions comprising reserved wells for control materials and also duplicate wells;
- Fig. 3D: a further particular embodiment of an allocation template illustrating a particular allocation sequence;
- Fig. 4A: a sample order (patient) complete prioritized sample distribution strategy applied onto the allocation template of figure 3A;
- Fig. 4B: an analyte/ test complete prioritized sample distribution strategy applied onto an allocation template;
- Fig. 4C: a first in first out sample distribution strategy applied onto the allocation template of figure 3D;
- Fig. 5A: a particular embodiment of the disclosed user interface showing a visual representation of the test order list and a visual representation of an allocation template before generation of the distribution list;
- Fig. 5B: a particular embodiment of the disclosed user interface showing a visual representation of the test order list; a visual representation of an allocation template and a visual representation of the distribution list;
- Fig. 6: a particular embodiment of the disclosed laboratory system with a pipettor; a control and a user interface;
- Fig. 7: a further embodiment of the disclosed laboratory system with a pipettor and a control unit comprised by an automated laboratory instrument and a user interface provided by a discrete computing device; and
- Fig. 8: an even further embodiment of the laboratory system wherein the user interface, the pipettor and the control unit are comprised by an automated laboratory instrument.

Note: The figures are not drawn to scale, are provided as illustration only and serve only for better understanding but not for defining the scope of the invention. No limitations of any features of the invention should be inferred from these figures.

### DETAILED DESCRIPTION

The use of the "a" or "an" are employed to describe elements and components of the embodiments herein. This is done merely for convenience and to give a general sense of the inventive concepts. This description should be read to include one or at least one and the singular also includes the plural unless it is obvious that it is meant otherwise.

As used herein qualifiers such as "about," "approximately," and "substantially" are intended to signify that the item or value being qualified is not limited to the exact value or amount specified, but includes some slight variations or deviations therefrom, caused by measuring error or imprecision, manufacturing tolerances, stress exerted on various parts, wear and tear, and combinations thereof, for example.

As used herein any reference to "one embodiment" or "an embodiment" means that a particular element, feature, structure, or characteristic described in connection with the embodiment is included in at least one embodiment. The appearances of the phrase "in one embodiment" in various places in the specification are not necessarily all referring to the same embodiment.

Certain terms will be used in this patent application, the formulation of which should not be interpreted to be limited by the specific term chosen, but as to relate to the general concept behind the specific term.

The term 'laboratory instrument' as used herein encompasses any apparatus or apparatus component operable to execute one or more processing steps / workflow steps on one or more biological samples. The expression 'processing steps' thereby refers to physically executed processing steps such as centrifugation, aliquotation, sample distribution, sample analysis and the like. The term 'instrument' covers preanalytical instruments, post-analytical instruments and also analytical instruments.

The term 'analyzer' /'analytical instrument' as used herein encompasses any apparatus or apparatus component configured to obtain a measurement value. An analyzer is operable to determine via various chemical, biological, physical, optical or other technical procedures a parameter value of the sample or a component thereof. An analyzer may be operable to measure said parameter of the sample or of at least one analyte and return the obtained measurement value. The list of possible analysis results returned by the analyzer comprises, without limitation, concentrations of the analyte in the sample, a digital (yes or no) result indicating the existence of the analyte in the sample (corresponding to a concentration above the detection level), optical parameters, DNA or RNA sequences, data obtained from mass spectroscopy of proteins or metabolites and physical or chemical parameters of various types. An analytical instrument may comprise units assisting with the pipetting, dosing, and mixing of samples and/or reagents. The analyzer may comprise a reagent holding unit for holding reagents to perform the assays. Reagents may be arranged for example in the form of containers or cassettes containing individual reagents or group of reagents, placed in appropriate receptacles or positions within a storage compartment or conveyor. It may comprise a consumable feeding unit. The analyzer may comprise a process and detection system whose workflow is optimized for certain types of analysis. Examples of such analyzer are clinical chemistry analyzers, coagulation chemistry analyzers, immunochemistry analyzers, urine analyzers, nucleic acid analyzers, used to detect the result of chemical or biological reactions or to monitor the progress of chemical or biological reactions.

The term 'post-analytical instrument' as used herein encompasses any apparatus or apparatus component that is configured to perform one or more post-analytical processing steps / workflow steps comprising - but not limited to - sample unloading, transport, recapping, decapping, temporary storage/ buffering, archiving (refrigerated or not), retrieval and/ or disposal.

The term 'pre-analytical instrument' as used herein encompasses any apparatus or apparatus component that is configured to perform one or more pre-analytical processing steps / workflow steps comprising - but not limited to - centrifugation, resuspension (e.g. by mixing or vortexing), capping, decapping, recapping, sorting, tube type identification, sample quality determination and/or aliquotation steps. Said processing steps may also comprise adding chemicals or buffers to a sample, concentrating a sample, incubating a sample, and the like.

An 'analysis system' as used herein comprises a control unit operatively coupled to one or more analytical; pre- and post-analytical work cells wherein the control unit is operable to control the work cells. In addition, the control unit may be operable to evaluate and/or process gathered analysis data, to control the loading, storing and/or unloading of samples to and/or from any one of the analyzers, to initialize an analysis or hardware or software operations of the analysis system used for preparing the samples, sample tubes or reagents for said analysis and the like.

The terms 'sample', 'patient sample' and 'biological sample' refer to material(s) that may potentially contain an analyte of interest. The patient sample can be derived from any biological source, such as a physiological fluid, including blood, saliva, ocular lens fluid, cerebrospinal fluid, sweat, urine, stool, semen, milk, ascites fluid, mucous, synovial fluid, peritoneal fluid, amniotic fluid, tissue, cultured cells, or the like. The patient sample can be pretreated prior to use, such as preparing plasma from blood, diluting viscous fluids, lysis or the like. Methods of treatment can involve filtration, distillation, concentration, inactivation of interfering components, and the addition of reagents. A patient sample may be used directly as obtained from the source or used following a pretreatment to modify the character of the sample. In some embodiments, an initially solid or semi-solid biological material can be rendered liquid by dissolving or suspending it with a suitable liquid medium. In some embodiments, the sample can be suspected to contain a certain antigen or nucleic acid.

The term 'quality control' or 'analytical quality control' refers to all those processes and procedures designed to ensure that the results of laboratory analysis (analytical tests) are consistent, comparable, accurate and within specified limits of precision.

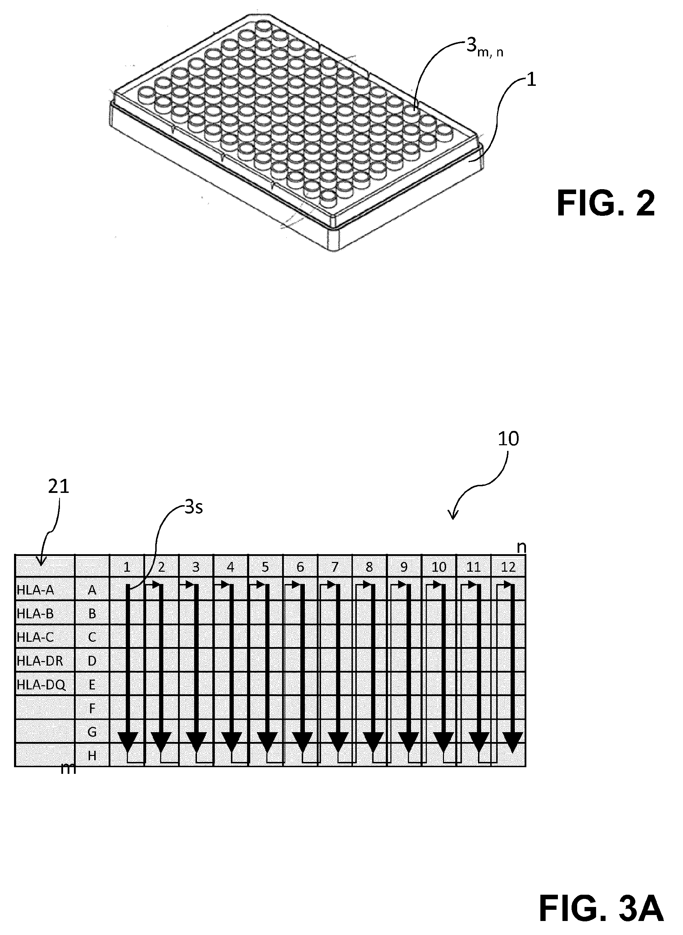

The term 'microplate' or 'microwell plate' as used herein refers to a plate / tray as commonly used in many in analytical research and clinical diagnostic testing laboratories having a plurality of sample wells arranged in a rectangular matrix. Each well of a microplate typically holds between tens of nanolitres to several milliliters of biological sample. Microplates have various dimensions, formats and configurations. For colorimetric immunoassays the microplate plate is commonly formed from a light transmitting plastic since reading of the assay results is typically done through the contents in the wells. In the case of photon emitting immunoassays, the microplate plates may be made of opaque plastic, such as black or white polystyrene, in order to reduce "cross-talk" in photometrically reading the results from well to well (i.e. to reduce interference caused by stray photons). A particular microplate 3 is illustrated on figure 2, with 96 wells 3 arranged in 8 rows and 12 columns in a landscape orientation.

A 'test order' as used herein encompasses any data object, computer loadable data structure, modulated data representing such data being indicative of one or more analytical tests to be executed on a particular biological sample or aliquot(s) thereof. For example, a test order may be a file or an entry in a database. A test order can indicate an analytical test if, for example, the test order comprises or is stored in association with an identifier of an analytical test to be executed on a particular sample.

The term 'analyte' is a component of a sample to be analyzed, e.g. molecules of various sizes, ions, proteins, metabolites and the like. Information gathered on an analyte may be used to evaluate the impact of the administration of drugs on the organism or on particular tissues or to make a diagnosis. Thus 'analyte' is a general term for substances for which information about presence and/or concentration is intended. Examples of analytes are e.g. glucose, coagulation parameters, endogenic proteins (e.g. proteins released from the heart muscle), metabolites, nucleic acids and so on.

The term 'analytical data' as used herein encompasses any data that is descriptive of a result of a measurement of a biological sample. In case of a calibration the analytical data comprises the calibration result, i.e. calibration data. In particular, the analytical data comprises an identifier of the sample for which the analysis has been performed and data being descriptive of a result of the analysis, such as measurement data.

The term 'analysis or 'analytical test' as used herein encompasses a laboratory procedure characterizing a parameter of a biological sample for qualitatively assessing or quantitatively measuring the presence or amount or the functional activity of an analyte.

The term 'control unit' as used herein encompasses any physical or virtual processing device configurable to control a laboratory instrument / or system comprising one or more laboratory instruments in a way that workflow(s) and workflow step(s) are conducted by the laboratory instrument / system. The control unit may, for example, instruct the laboratory instrument / system to conduct pre-analytical, post analytical and analytical workflow(s)/ workflow step(s). The control unit may receive information from a data management unit regarding which steps need to be performed with a certain sample. In some embodiments, the control unit might be integral with a data management unit, may be comprised by a server computer and/or be part of one laboratory instrument or even distributed across multiple instruments of the laboratory system. The control unit may, for instance, be embodied as a programmable logic controller running a computer-readable program provided with instructions to perform operations.

The term 'communication network' as used herein encompasses any type of wireless network, such as a WIFI, GSM, UMTS or other wireless digital network or a cable based network, such as Ethernet or the like. In particular, the communication network can implement the Internet protocol (IP). For example, the communication network comprises a combination of cable-based and wireless networks.

The term "pipettor" as used herein comprises a device (such as a liquid handling robot) for aspirating and for dispensing a volume of a sample or other liquid, such as reagents or diluting buffer, into a vessel, such as a reaction vessel, a microplate plate, etc. The pipettor may comprise one or more reusable washable needles such as a steel needle, or use disposable pipette tips. The pipettor may be mounted to a transfer head that can be moved in one or two directions of travel in a plane, e.g., with guiding rails and a third direction of travel orthogonal to the plane, with a spindle drive or the like. For instance, the pipettor may be moved horizontally between a primary sample tube and a vessel, and vertically in order to withdraw or dispense the liquid biological sample or other liquids. The pipettor may be integrated, i.e. built in a work-cell or be a module of the system operatively connected to a work-cell.

The term "liquid" as used herein relates to any type of liquid which has to be transferred during an analytical process. Thus, the term includes liquid samples. It also includes reagents / suspensions of reagents, control or calibration material.

Reagents necessary for performing the analysis of analytes include reagents for sample preparation, control reagents, reagents for reacting with the analyte to obtain a detectable signal, and/or reagents necessary for detecting the analyte. Such reagents may include reagents for isolating an analyte and/or reagents for processing a sample and/or reagents for reacting with an analyte to obtain a detectable signal and/or washing reagents and/or diluents.

The term "RFID tag" as used herein refers to either an active or passive RFID tag that contains information. An RFID tag or transponder includes a coil or antenna and some information stored on an RFID chip that can be read and/or written by an RFID reader. Correspondingly the RFID tag can be read only or read/write and the information associated with the RFID tag can be hard-coded into the RFID tag at the time of manufacture or at some later time.

The term "RFID reader" as used herein includes devices that can read information from and/or write information into an RFID tag. Typically, RFID readers can include a coil or antenna and circuitry to transmit and receive signals with the coil or antenna. The RFID reader antenna generates an electromagnetic field, thereby transferring energy to the tag. Depending on the design of the tag, a portion of the energy transferred to the tag will be reflected to the reader so as to provide information about the tag back to the reader.

The term 'user interface' as used herein encompasses any suitable piece of software and/or hardware for interactions between an operator and a machine, including but not limited to a graphical user interface for receiving as input a command from an operator and also to provide feedback and convey information thereto. Also, a system/ device may expose several user interfaces to serve different kinds of users/ operators.

The term 'workflow' as used herein encompasses any task that comprises a number of steps, such as for maintenance or operation of the system or one of its system components.

The term 'workflow step' as used herein encompasses any activity belonging to a workflow. The activity can be of an elementary or complex nature and is typically performed at or by means of one or more work cell(s).

A unit being 'operatively coupled' to an apparatus is a unit which is part of said apparatus or is operable to exchange data with said apparatus via a network connection, e.g. an intranet or internet.

In the following, aspects of embodiments of the disclosed method / system shall be described with reference to the figures provided as illustration only and serving a better understanding but not for defining the scope of the invention. In these figures, method steps and functional/ structural features according to only particular embodiments of the disclosed method / system are illustrated with dashed lines. However, the usage of dashed lines should not be interpreted as diminishing the importance, the technical effects or advantages conferred by such features.

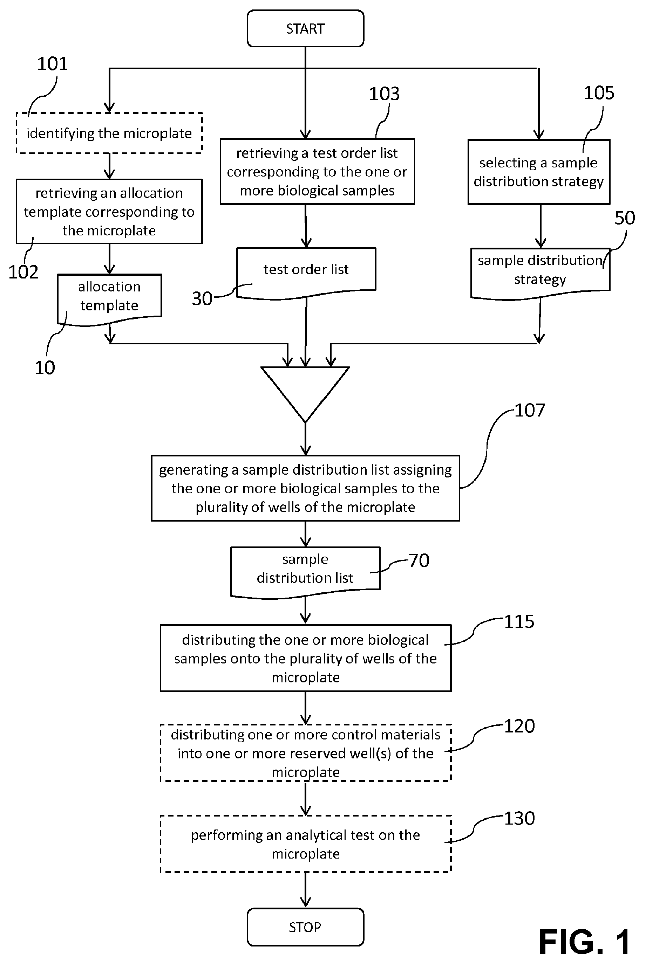

As illustrated on figure 1, in one aspect, a method for distributing one or more biological samples to a plurality of wells 3 of a microplate 1 comprises the steps of:
- retrieving 102 an allocation template 10 corresponding to the microplate 1;
- retrieving 103 a test order list 30 comprising a plurality of test orders corresponding to the one or more biological samples;
- selecting 105 a sample distribution strategy 50 from one or more sample distribution strategies;
- generating 107 a sample distribution list 70 assigning the one or more biological samples to the plurality of wells 3 of the microplate 1 according to the test order list 30, the allocation template 10 and the selected sample distribution strategy 50; and
- distributing 115 the one or more biological samples onto the plurality of wells 3 of the microplate 1 according to the sample distribution list 70.

It shall be noted, that according to embodiments of the disclosed method, the steps of retrieving 102 the allocation template 10; selecting 105 the sample distribution strategy 50 and retrieving 103 the test order list 30 may be performed simultaneously or sequentially. According to further aspects of the disclosed method/ system, steps 102, 103 and 105 are interdependent. For example a certain sample distribution strategy 50 can be applied only to a particular allocation template 10 which is requested by a test order list 30.

According to particular embodiments herein disclosed, the step of retrieving 102 an allocation template 10 corresponding to the microplate 1 is preceded by the step of identifying the microplate 1 based on an identification tag 5 associated with the microplate 1, such as an RFID tag or barcode label using an identification tag reader 230 such as an RFID reader or barcode reader.

The allocation template 10 is retrieved from a computer readable storage medium, such as a hard drive, a solid state disk, a volatile or non-volatile memory. The allocation template 10 may be retrievable directly or via a communication network 260. Alternatively or in addition, the allocation template 10 may be retrievable from the identification tag 5 itself, in particular from an RFID tag.

According to particular embodiments herein disclosed, allocation template 10 defines one or more of the following:
- dimensions of the microplate 1 including the number of rows m and number of columns n in which the wells 3 of the microplate 1 are arranged;
- orientation of the microplate 1 such as landscape or portrait orientation;
- format of the microplate 1, such as a specific format for a particular instrument or instrument type (e.g. Roche LightCycler®);
- start well 3s of the microplate 1 identifying the first well to be allocated a sample;
- allocation sequence 13 of the microplate 1 defining an order in which the wells 3 of the microplate 1 are to be allocated;
- assignment restrictions 20 of the microplate 1.

Particular allocation templates 10 shall be described with reference to figures 3A through 3D.

According to particular embodiments herein disclosed, assignment restriction(s) 20 of the allocation template 10 identify one or more wells 3 of the microplate 1 as:
- dedicated well(s) 21 for particular analyte(s)/ analytical test(s);
- reserved well(s) 22 for particular control(s) (e.g. negative control);
- duplicate well(s) 23 for receiving duplicate(s) of sample(s) in other well(s) of the microplate 1;
- excluded/blank well(s) 24 which are not to be assigned; or
- freely assignable well(s) which can hold any test analyte, any control material, a duplicate of a sample in other well(s) or be left as blank.

Corresponding to the one or more biological samples, a test order list 30 is retrieved 103. According to various embodiments, the test order list 30 may be retrieved from a computer readable storage medium, such as a hard drive, a solid state disk, a volatile or non-volatile memory. The test order list 30 may be retrievable directly or via a communication network 260 from a Laboratory Information System LIS and/or a Hospital Information System HIS 400. Alternatively or in addition, the test order list 30 may be provided via the user interface 250.

As shown on figure 1, a further step of the disclosed method for distributing one or more biological samples to a plurality of wells 3 of a microplate 1 comprises selection of a sample distribution strategy 50 from one or more sample distribution strategies. In general terms, a sample distribution strategy defines how test orders corresponding to the biological sample are selected for assignment to wells 3 of a microplate 1.

According to embodiments of the disclosed method/ system, a sample distribution strategy 50 defines how test orders are grouped and/or filtered and/ or sorted and/or prioritized for assignment.

The term 'grouping' with reference to grouping of test orders, means ion this context forming groups of test orders based on one or more criteria - such as the test orders belonging to the same sample (or patient), the same analytical test, originating from the same laboratory, etc. By grouping test orders sample distribution strategies 50 aims to ensure that test orders fulfilling the particular criteria by which they have been grouped are assigned one after the other, increasing the chances these will be assigned to the same (or subsequent) microplates and hence be processed together (or within a short time-span). For example by grouping test orders by origin (such as laboratory), a sample distribution strategy 50 aims to ensure that samples from that laboratory are distributed to the same (or subsequent) microplates 1. On the other hand, by grouping test orders by the analytical test to be performed, the sample distribution strategy 50 aims to ensure that samples for the same analytical test are assigned to microwell plate grouped together instead of dispersed randomly.

The term 'filtered' in this context means an exclusive selection of one or more test orders based on a certain criteria, such as the test orders belonging to the same sample (or patient), the same analytical test, originating from the same laboratory, etc. Filtering of test orders may be a part of grouping.

Sorting of the test orders refers to changes in the sequence of the test orders in the test order list 30. Sorting of the test orders may be a means to achieve a prioritization but also part of grouping.

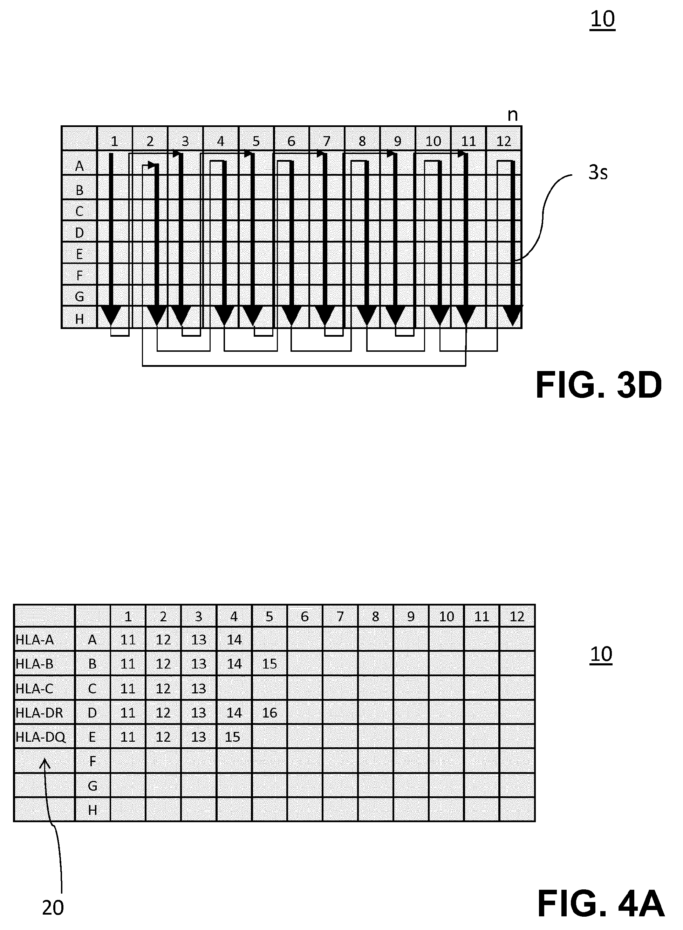

Particular sample distribution strategies 50 applied onto allocation templates 10 shall be described with reference to figures 4A through 4C.

As a next step, as shown in figure 1, a sample distribution list 70 assigning the one or more biological samples to the plurality of wells 3 of the microplate 1 is generated. The assignment of the one or more biological samples to the plurality of wells 3 of the microplate 1 is performed in that the test orders of the test order list 30 are being selected/ prioritized / sorted / filtered according to the selected sample distribution strategy 50 and distributed to the plurality of wells 3 of the microplate 1 according to the allocation template 10.

Thus, by means of the sample distribution strategy 50 the test order assignment adapts to the specific requirements of a particular laboratory, while the distribution of the samples into wells 3 is performed according to the allocation template 10, thereby adapting to the particular microplate 1 used.

After the sample distribution list 70 has been generated, the one or more biological samples (or aliquots thereof) are distributed onto the plurality of wells 3 of the microplate 1 according to the sample distribution list 70. The wording distributing with respect to a biological sample shall be understood to comprise distributing the entire sample volume but also distributing aliquots of the biological sample.

According to embodiments of the disclosed method / system, the biological samples are distributed onto the plurality of wells 3 of the microplate 1 according to the sample distribution list 70 automatically or semi-automatically by a pipettor 210 of an automated laboratory instrument 300.

According to embodiments of the disclosed method / system - in addition to distributing the biological samples - one or more control materials are distributed in a step 120 into one or more reserved well(s) 3 of the microplate 1 according to the assignment restrictions of the allocation template 10 corresponding to the microplate 1. Control materials, such as positive and negative controls serve the purpose of providing evidence that each analytical test is successfully performed and is giving the expected level of sensitivity and specificity as characterized during technical optimization and validation of the analytical test for diagnostic use. Positive controls primarily monitor calibration of the system and sensitivity. Negative controls are primarily used to evaluate the specificity of the analytical tests to identify false-positive results.

According to embodiments of the disclosed method, after the samples and as the case may be control materials have been distributed into the wells 3 of the microplate 1, an analytical test is performed on the microplate 1.

Particular allocation templates 10 shall be described now with reference to figures 3A through 3D for a microplate 1 with 96 wells arranged in 8 rows and 12 columns in a landscape orientation.

Figure 3A shows one example of an allocation template 10 which defines:
- number of wells 3 to be equal to 96;
- the number of rows m equal to 8 (A to H);
- the number of columns n equal to 12 (1 to 12);
- orientation: landscape;
- well A1 as the start well 3s; and
- the allocation sequence 13 as:
   o starting from the first row and first column, i.e. well_{A1};
   o allocating all wells of a column, that is well_{A1} to well_{H1;}
   o allocating all wells (well A2 to H2) of the next column; and
- the assignment restriction(s) 20 to comprising:
   o dedicated wells for various human leukocyte HLA analytes
      ▪ wells A1 through A12 as dedicated well(s) 21 for HLA-A;
      ▪ wells B1 through B12 as dedicated well(s) 21 for HLA-B;
      ▪ wells C1 through C12 as dedicated well(s) 21 for HLA-C;
      ▪ wells D1 through D12 as dedicated well(s) 21 for HLA-D; and
      ▪ wells E1 through E12 as dedicated well(s) 21 for HLA-DQ.

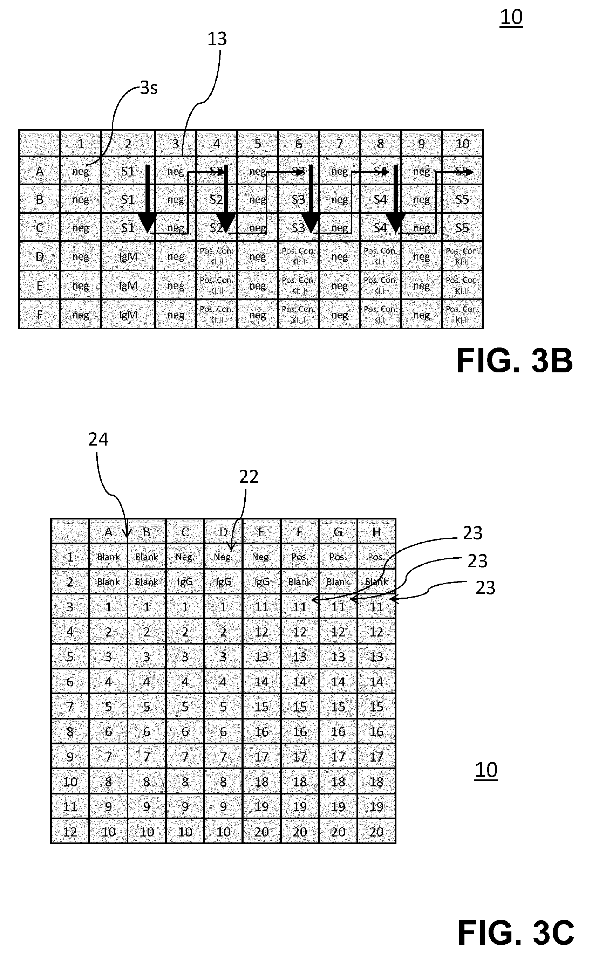

Figure 3B shows a further embodiment of an allocation template 10 which defines:
- number of wells 3 to be equal to 60;
- the number of rows m equal to 6 (A to F);
- the number of columns n equal to 10 (1 to 10);
- orientation: landscape;
- well A2 as the start well 3s; and
- the allocation sequence 13 as illustrated by arrows 13:
   o wells A2 through C2;
   o wells A4 through C4;
   o wells A6 through C6;
   o wells A8 through C8;
   o wells A10 through C10;
- the assignment restriction(s) 20 comprising:
   o wells of odd columns 1, 3, 5, 7 and 9 as reserved wells 22 for negative control;
   o wells of odd columns as reserved well(s) 22 for negative control;
   o wells D6 through F6, D8 through F8 and D10 through F10 as reserved wells 22 for positive control(s); and
   o the second and third rows of each even column as duplicate wells 23, e.g. wells B2 and C2 aliquots (duplicates) of the sample in A2.

Figure 3C shows an even further embodiment of an allocation template 10 which defines:
- number of wells 3 to be equal to 96;
- the number of rows m equal to 12 (1 to 12);
- the number of columns n equal to 8 (A to H);
- orientation: portrait;
- well A3 as the start well 3s; and
- the allocation sequence 13 as illustrated by the numbering of wells on the figure;
- the assignment restriction(s) 20 comprising:
   o wells C1 and D1 reserved wells 22 for negative control;
   o wells F1 to H1 reserved wells 22 for positive control;
   o wells A1, B1, A2, B2 and F2 to H2 as blank wells 24;
   o every second, third and fourth well as duplicate well 23.

Figure 3D shows one example of an allocation template 10 similar to that on figure 3A, but wherein the allocation sequence 13 defines that first the odd columns are assigned and only thereafter are the even numbered columns - as illustrated with the multi-segment arrow 3s.This allocation template 10 is advantageous for example if a 48 channel sequencer is used which dives into every second column of a 96 well microplate 1, the distribution being performed in two stages if there are more than 48 samples (sample aliquots) to be distributed.

It shall be noted that the allocation templates 10 presented here are particular examples and many other allocation templates 10 can be configured based on different microplates but also according to the specific analytical test as well as differing regulatory requirements. It shall also be noted that a multitude of different allocation templates 10 can exist for the same microplate with a particular physical configuration.

Particular sample distribution strategies 50 applied onto allocation templates 10 shall be now described with reference to figures 4A through 4C.

Figure 4A illustrates a sample order (patient) complete prioritized strategy 50 applied onto the allocation template 10 of figure 3A. According to the sample complete order prioritized strategy, test orders corresponding to the same biological sample (same patient) are grouped and/or filtered and/ or sorted and/or prioritized for assignment. Thus according to the sample order (patient) complete prioritized strategy 50 - as illustrated on figure 4A -the test orders (from the test order list 30) are grouped by sample (in this example all test orders for sample nr. 11 are grouped together). Then the test orders within these groups (in this example the test orders from the group of sample nr. 11) are sorted by the analytical test to be performed (in this example in the order HLA-A, -B, -C, -DR and -DQ). Hence all test orders (HLA-A, -B, -C, -DR and -DQ) of sample nr. 11 are assigned first to wells 3 of the microplate 1 so as to prioritize all tests of sample nr. 11 before the next sample is assigned. After selection of the samples to be distributed, assignment is performed according to the allocation template 10, which defines both the allocation sequence 13 but also the dedicated wells 21 where aliquots of the sample nr. 11 for each corresponding test order are assigned to. For example the test order for sample nr. 11 for HLA-A is assigned to well A1 followed by the test order for HLA-B for the same sample nr. 11 in the next well (next in sequence according to the strategy and dedicated for this test) according to the allocation template 10, namely well B1. Aliquots of the next groups of test orders (for samples nr. 12 and 13) for which test orders for all HLA-A through DQ tests are in the test order list 30 are assigned in a similar manner. In accordance with the sample order (patient) complete prioritized strategy 50, the next sample to be assigned is sample nr. 14 with corresponding test orders for HLA-A, B and DR which are assigned to wells A4, B4 and D4 respectively. Aliquots of sample nr. 15 for tests HLA-B and DQ are assigned next to wells B5 and E5. Sample nr. 16 with a test order only for HLA-DR is assigned to well D5 as the next available well for HLA-DR according to the allocation template 10 for this microplate 1.

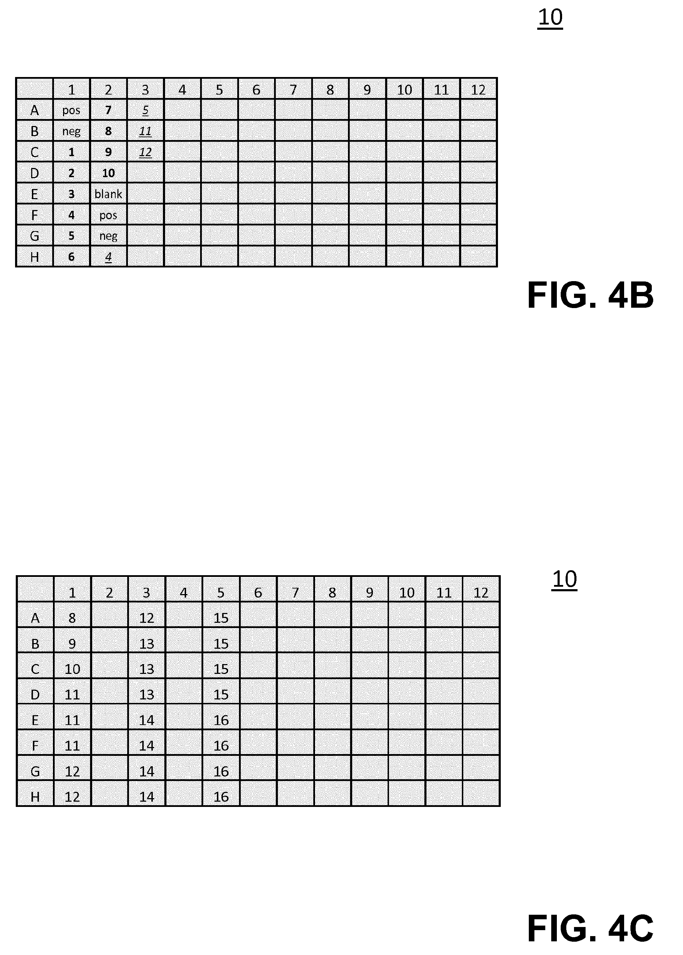

Figure 4B illustrates an analyte/ test complete prioritized strategy 50 applied onto the allocation template 10 of figure 3A with the additional assignment restriction 20 that test orders corresponding to the same analytical test (same analyte) are separated by a blank well 24, a reserved well 22 for a positive and a reserved well 22 for a negative control material.

As illustrated on figure 4B, according to the analyte/ test complete prioritized strategy 50, test orders for the same analytical test (same analyte) are grouped together. On this figure samples (or sample aliquots) with test orders for a first analytical test (e.g. HIV) are illustrated with bold characters while samples (or sample aliquots) with test orders for a second analytical test (e.g. CMV) are illustrated with italic-underlined characters. Thus, the assignment of the one or more biological samples to the plurality of wells 3 of the microplate 1 according to the test order list 30, the allocation template 10 and according to the analyte/ test complete prioritized strategy 50 is performed in that the test orders for the same analytical test (same analyte) are grouped together and then the groups are sorted so that all samples with a test order for the first analytical test are assigned followed by assignment of samples with a test order for the second analytical test are assigned and so on.

Figure 4C illustrates a first in first out FIFO sample distribution strategy 50 applied onto the allocation template 10 of figure 3D, wherein the test orders are sorted so that samples are assigned to the wells 3 of the microplate 1 in the same order as the corresponding test orders have been registered onto the test order list 30.

Further embodiments of the sample distribution strategy 50 include an origin complete prioritized strategy 50, wherein test orders with same origin - such as same laboratory/ hospital / medical practice - are grouped and/or filtered and/ or sorted and/or prioritized for assignment to ensure all analytical test ordered by the same laboratory/ hospital / medical practice are available substantially at the same time.

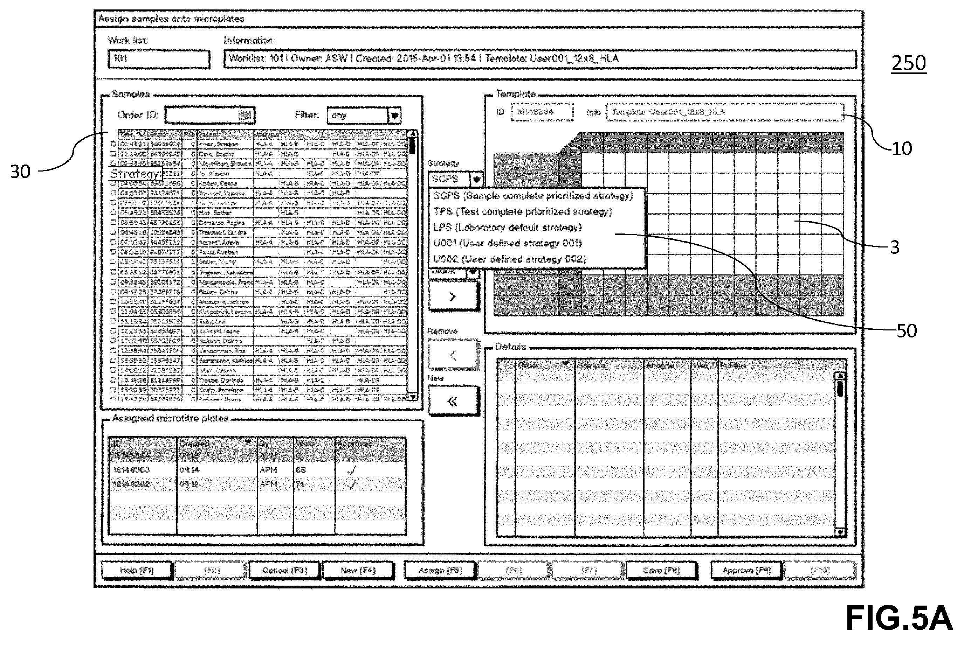

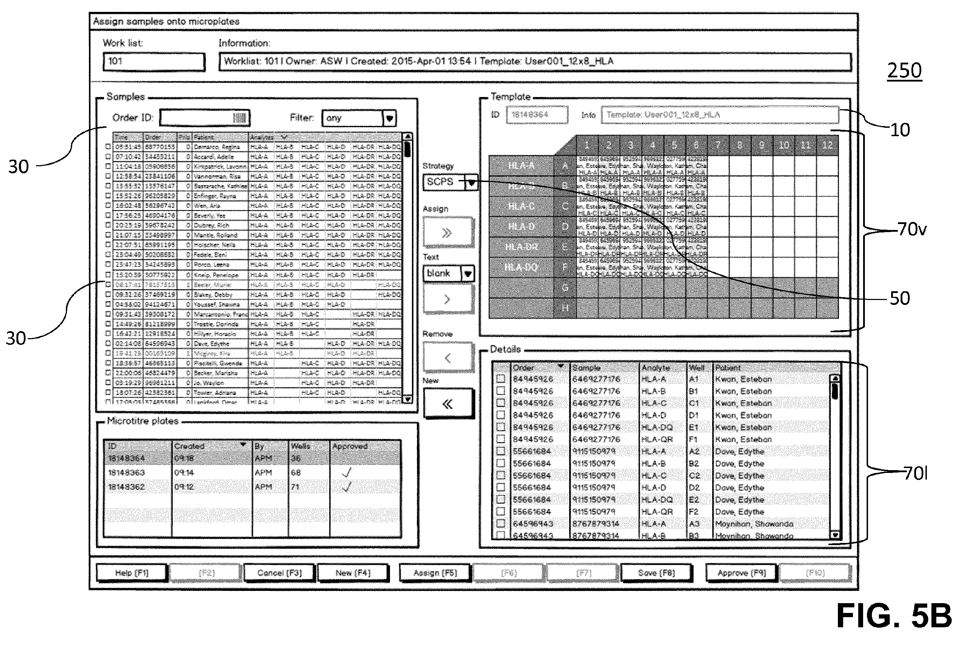

Particular embodiments of a user interface 250 shall be described with reference to figures 5A and 5B.

Figure 5A shows a user interface 250 with - among other elements - a visual representation of the test order list 30 and a visual representation of an allocation template 10. On figure 5A, the allocation template 10 is shown before generation of the distribution list 70, the wells 3 of the microplate 1 being shown empty with no samples assigned yet. According to particular embodiments, the user interface 250 provides both the option to generate the sample distribution list 70 automatically (that is according to the test order list 30), the allocation template 10 and the selected sample distribution strategy 50) but also provide the operator the option to assign samples to microplate 1 wells 3 individually. This is advantageous as it provides the benefits of automatic sample assignment combined with flexibility for the operator to handle exceptions.

Furthermore, according to embodiments, test orders of the test order list 30 can be labelled as 'Prio' orders, which are given priority in the assignment. Prioritized samples are also called STAT samples. A 'STAT sample' is a sample which needs to be processed and analyzed very urgently as the analysis result may be of life-crucial importance for a patient. According to embodiments of the disclosed method / system, STAT samples are given priority regardless of the sample distribution strategy 50 selected.

Figure 5B shows the user interface 250 of figure 5A after generation of the distribution list 70. In the embodiment shown on figure 5B, the distribution list 70 is visually represented as a distribution table 70v on the user interface 150 by showing the order number of the samples in the visual representation of the corresponding well 3 of the microplate 1. In other words, the visual representation of the allocation template 10 is filled in with the order information for the biological samples according to the sample distribution list 70. Alternatively or in addition, the sample distribution list 70 may be shown directly as a listing 70l on the user interface 250, such being advantageous for microplates with a high number of wells.

The user interface 250 according to embodiments herein disclosed is advantageous as it allows a what you see is what you get WYSIWYG visualisation of allocation templates 10 and the microplate 1 before and after distribution of the samples into its wells 3.

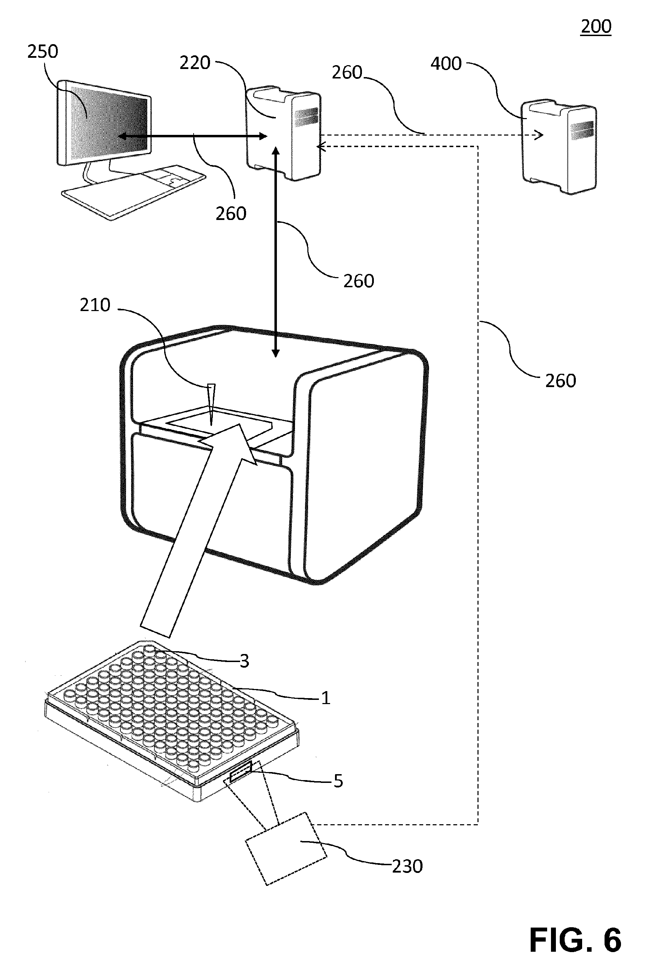

Embodiment of a disclosed laboratory system 200 shall now be described with reference to figures 6 through 8.

Figure 6 shows a laboratory system 200 comprising a pipettor 210 configured for dispensing a volume of biological sample into one or more wells 3 of a microplate 1; a user interface 250 configured to receive a selection of a sample distribution strategy 50 from one or more distribution strategies; and a control unit 220. According to the embodiment illustrated on this figure, the pipettor 210, the user interface 250 and the control unit 220 are discrete hardware units communicatively connected by a communication network 260. According to embodiments of the disclosed a laboratory system 200, the pipettor 210 is part of an automated liquid handler having a robotic arm displaceable in the x and y directions of the Cartesian coordinate system while the pipettor 210 itself can be displaced in the z-direction such as to aspirate and then dispense a volume of a sample into a well 3 of the microplate 1.

According to further embodiments, the laboratory system 200 further comprises an identification tag reader 230 - such as an RFID reader or barcode reader - configured to read an identification tag 5 - such as an RFID tag or barcode label - associated with the microplate 1, while the control unit 220 is configured to retrieve the allocation template 10 corresponding to the microplate 1 based on the identification tag 5.

According to further embodiments, the laboratory system 200, in particular the control unit 220 is communicatively connected to a Laboratory Information System LIS and/or a Hospital Information System HIS 400, the control unit 220 being configured to retrieve from the Laboratory Information System LIS and/or a Hospital Information System HIS 400 one or more of the following:
- the allocation template 10 corresponding to the microplate 1;
- the test order list 30 corresponding to the one or more biological samples; and
- the one or more distribution strategies.

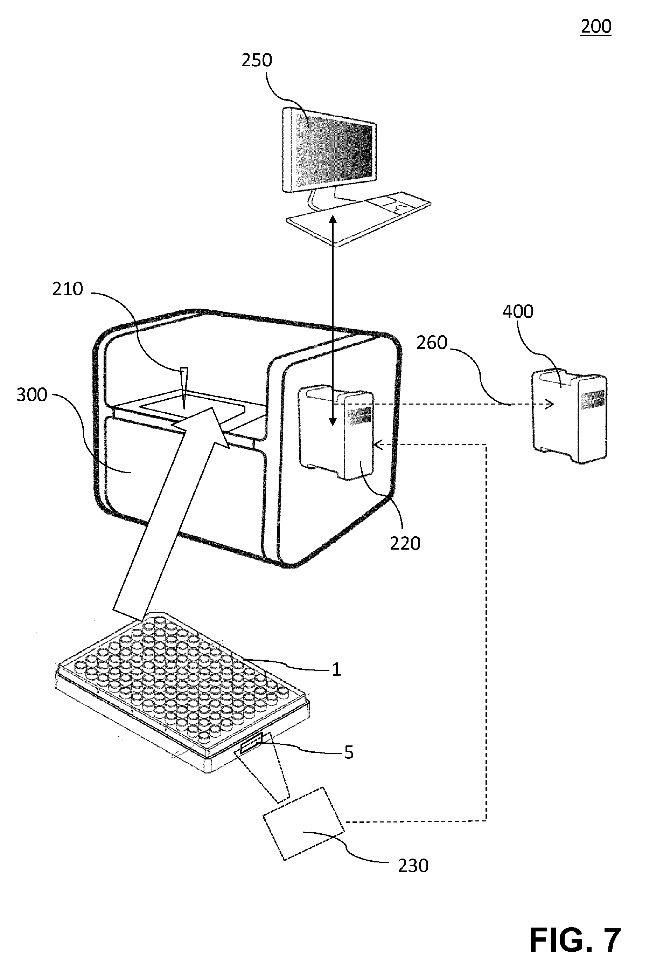

Figure 7 shows a further embodiment of the laboratory system 200 wherein the pipettor 210 and the control unit 220 are comprised by an automated laboratory instrument 300 while the user interface 250 is provided by a discrete computing device and wherein the sample distribution list 70 is transmitted to the control unit 220 via a communication network 260.

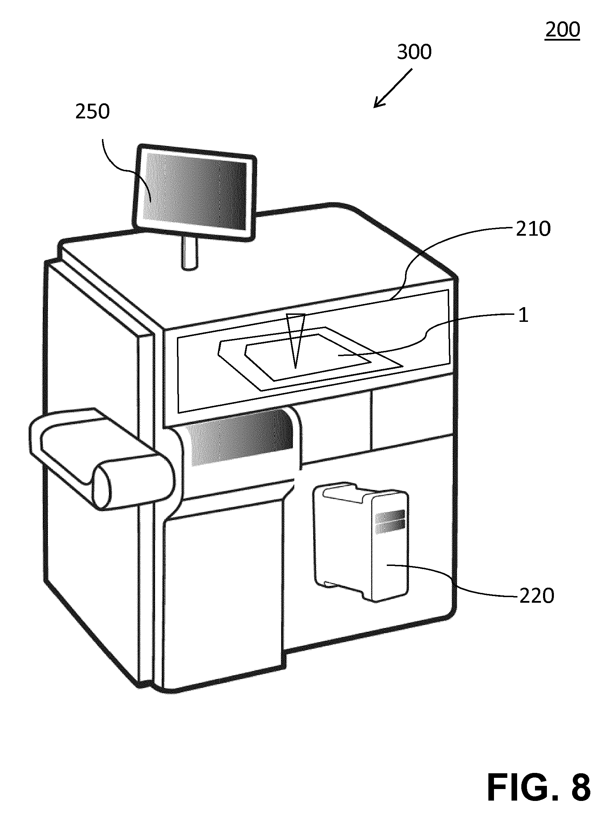

Figure 8 shows an even further embodiment of the laboratory system 200 wherein the user interface 250, the pipettor 210 and the control unit 220 are comprised by an automated laboratory instrument 300.

In a further aspect of the disclosed laboratory system 200, the control unit 220 is further configured to control the automated laboratory instrument 300 to performing an analytical test on the microplate 1 after the one or more biological samples have been distributed onto the plurality of wells 3 of the microplate 1.

Further disclosed and proposed is a computer program including computer-executable instructions for performing the disclosed method in one or more of the embodiments enclosed herein when the program is executed on a computer or computer network. Specifically, the computer program may be stored on a computer-readable data carrier. Thus, specifically, one, more than one or even all of method steps may be performed by using a computer or a computer network, preferably by using a computer program.

Further disclosed and proposed is a computer program product having program code means, in order to perform the disclosed method in one or more of the embodiments enclosed herein when the program is executed on a computer or computer network. Specifically, the program code means may be stored on a computer-readable data carrier. The product may generally exist in an arbitrary format, such as in a paper format, or on a computer-readable data carrier. Specifically, the computer program product may be distributed over a data network.

Further disclosed and proposed is a data carrier having a data structure stored thereon, which, after loading into a computer or computer network, such as into a working memory or main memory of the computer or computer network, may execute the disclosed method according to one or more of the embodiments disclosed herein.

As used herein, a computer program product refers to the program as a tradable product.

Further disclosed and proposed is a modulated data signal which contains instructions readable by a computer system or computer network, for performing the method according to one or more of the embodiments disclosed herein.

Referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Furthermore, hereby disclosed and proposed are:
- A computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

It will be understood that many variations could be adopted based on the specific structure hereinbefore described without departing from the scope of the invention as defined in the following claims.

**REFERENCE LIST:**

| | |
|---|---|
| microplate | 1 |
| wells (of the microplate) | 3 |
| identification tag | 5 |
| number of rows (of wells of the microplate) | m |
| number of columns (of wells of the microplate) | n |
| start well (of the microplate) | 3s |
| allocation template | 10 |
| allocation sequence | 13 |
| assignment restriction | 20 |
| dedicated well | 21 |
| reserved well | 22 |
| duplicate well | 23 |
| excluded/blank well | 24 |
| identifying the microplate | 101 |
| retrieving an allocation template | 102 |
| sample distribution strategy | 50 |
| selecting a sample distribution strategy | 105 |
| test order list | 30 |
| retrieving a test order list | 103 |
| sample distribution list | 70 |
| table-based visual representation of the sample distribution list | 70v |
| visual representation of the sample distribution list | 70l |
| generating a sample distribution list | 107 |
| distributing control material into one or more reserved wells | 120 |
| performing an analytical test on the microplate | 130 |
| laboratory system | 200 |
| pipettor | 210 |
| control unit | 220 |
| identification tag reader | 230 |
| user interface | 250 |
| communication network | 260 |
| automated laboratory instrument (300) | 300 |
| Laboratory Information System LIS and/or a Hospital Information System HIS | 400 |

## Claims

1. A method for distributing one or more biological samples to a plurality of wells (3) of a microplate (1), the method comprising:
- retrieving (102) an allocation template (10) corresponding to the microplate (1);
- retrieving (103) a test order list (30) comprising a plurality of test orders corresponding to the one or more biological samples;
- selecting (105) a sample distribution strategy (50) from one or more sample distribution strategies;
- generating (107) a sample distribution list (70) assigning the one or more biological samples to the plurality of wells (3) of the microplate (1) according to the test order list (30), the allocation template (10) and the selected sample distribution strategy (50); and
- distributing (115) the one or more biological samples onto the plurality of wells (3) of the microplate (1) according to the sample distribution list (70).

2. The method according to claim 1, wherein the allocation template (10) defines one or more of the following:
- dimensions of the microplate (1) including number of rows (m) and number of columns (n) in which the wells (3) of the microplate (1) are arranged;
- orientation of the microplate (1);
- format of the microplate (1);
- start well (3s) of the microplate (1) identifying the first well to be allocated a sample;
- allocation sequence (13) of the microplate (1) defining an order in which the wells (3) of the microplate (1) are to be allocated;
- assignment restriction(s) (20) of the microplate (1).

3. The method according to claim 2, wherein the assignment restriction(s) (20) of the allocation template (10) identify one or more wells (3) of the microplate (1) as:
- dedicated well(s) (21) for particular analyte(s) and/or analyte group(s);
- reserved well(s) (22) for particular control(s) (e.g. negative control);
- duplicate well(s) (23) for receiving duplicate(s) of sample(s) in other well(s) of the microplate (1);
- excluded/blank well(s) (24) which are not to be assigned; or
- freely assignable well(s) which can hold any test analyte, any control material, a duplicate of a sample in other well(s) or be left as blank.

4. The method according to one of the preceding claims, wherein the one or more distribution strategies define an order in which test orders of the test order list (30) are assigned to the plurality of wells (3) of the microplate (1).

5. The method according to claim 4, wherein the one or more sample distribution strategies comprise:
- sample complete prioritized strategy 50, wherein test orders corresponding to the same biological sample (same patient) are grouped and/or filtered and/ or sorted and/or prioritized for assignment;
- analyte/ test complete prioritized strategy 50, wherein test orders for the same analytical test (same analyte) are grouped and/or filtered and/ or sorted and/or prioritized for assignment;
- origin complete prioritized strategy 50, wherein test orders with same origin - such as laboratory/ hospital - are grouped and/or filtered and/ or sorted and/or prioritized for assignment.

6. The method according to claim 5, wherein groups of test orders corresponding to the same biological sample (same patient) and/ or groups of test orders for the same analytical test (same analyte) are separated by a blank well (24) and/or one or more reserved well(s) (22) for control material(s).

7. The method according to one of the preceding claims, wherein the one or more biological samples are distributed onto the plurality of wells (3) of the microplate (1) according to the sample distribution list (70) automatically by a pipettor (210) of an automated laboratory instrument (300).

8. The method according to one of the preceding claims, further comprising the step (101) of identifying the microplate (1) based on an identification tag associated with the microplate (1), wherein the allocation template (10) is retrieved based on the identification tag (5).

9. The method according to one of the claims 3 to 8 further comprising the step (120) of distributing one or more control materials into one or more reserved well(s) of the microplate (1) according to the assignment restriction(s) of the allocation template (10) corresponding to the microplate (1).

10. The method according to one of the preceding claims further comprising the step (130) of performing an analytical test on the microplate (1) after the one or more biological samples have been distributed onto the plurality of wells (3) of the microplate (1).

11. A laboratory system (200) comprising:
- a pipettor (210) configured for dispensing a volume of biological sample into one or more wells (3) of a microplate (1); and
- a user interface (250) configured to receive a selection of a sample distribution strategy (50) from one or more distribution strategies;
- a control unit (220);
wherein the control unit (220) is configured:
- to retrieve an allocation template (10) corresponding to the microplate (1);
- to retrieve a test order list (30) corresponding to the one or more biological samples;
- to generate a sample distribution list (70) assigning the one or more biological samples to the plurality of wells (3) of the microplate (1) according to the test order list (30), the allocation template (10) and the selected sample distribution strategy (50);
- to control the pipettor (210) such as to distribute the one or more biological samples onto the plurality of wells (3) of the microplate (1) according to the sample distribution list (70).

12. The laboratory system (200) of claim 11 further comprising an identification tag reader (230) configured to read an identification tag (5) associated with the microplate (1), wherein the control unit (220) is configured to retrieve the allocation template (10) corresponding to the microplate (1) based on the identification tag (5).

13. The laboratory system (200) of claim 11, wherein the control unit (220) is configured to retrieve the allocation template (10) corresponding to the microplate (1) based on a selection via the user interface (250).

14. The laboratory system (200) of one of the claims 11 to 13, wherein the user interface (250), the pipettor (210) and the control unit (220) are comprised by an automated laboratory instrument (300).

15. The laboratory system (200) of one of the claims 11 to 13, wherein the pipettor (210) and the control unit (220) are comprised by an automated laboratory instrument (300) while the user interface (250) is provided by a discrete computing device and wherein the sample distribution list (70) is stored on a computer readable medium accessible by the control unit (220) and/or sample distribution list (70) is transmitted to the control unit (220) via a communication network (260).

16. The laboratory system (200) of claim 14 or 15, wherein the control unit (220) is further configured to control the automated laboratory instrument (300) to performing an analytical test on the microplate (1) after the one or more biological samples have been distributed onto the plurality of wells (3) of the microplate (1).
